# EUROPEAN PATENT APPLICATION

(11) **EP 4 844 061 A1**
(43) Date of publication of application: **25.11.2026**
(21) Application number: 24926799.8
(22) Date of filing: 04.11.2024
(51) Int. Cl.: C12N 9/96, C12N 9/16

(54) **FREEZE-DRYING AUXILIARY MATERIALS, FREEZE-DRYING PRESERVATION SOLUTION AND USE THEREOF**

(30) Priority: 01.03.2024 CN 202410236318
(71) Applicant: Shenzhen Yhlo Biotech Co., Ltd., Shenzhen, Guangdong 518116 (CN)
(72) Inventor: WANG, Jingjing, Shenzhen, Guangdong 518116 (CN); QIAN, Chungen, Shenzhen, Guangdong 518116 (CN); HU, Kunhui, Shenzhen, Guangdong 518116 (CN)
(74) Representative: Michalski Hüttermann & Partner mbB
(86) International application number: PCT/CN2024/129685
(87) International publication number: WO 2025/179938

(57) **Abstract**

A freeze-drying excipient, a freeze-drying preservation solution, and use thereof are provided. The freeze-drying excipient includes a saccharide, an amino acid, and an auxiliary agent in a mass ratio of (4-20):(2-12):(3-15). The auxiliary agent includes one or more selected from the group consisting of polyvinylpyrrolidone, polyethylene glycol, mannitol, and bovine serum albumin. The saccharide and the amino acid at specific concentrations and the auxiliary agent of a specific type are selected for the freeze-drying excipient. The components cooperate with each other and act synergistically, so that when the prepared freeze-drying preservation solution is used for preserving the ALP enzyme, it can be stored in a sealed manner for more than 18 months under room temperature conditions, and maintains an enzyme activity retention rate above 87% after storage under an elevated-temperature condition of 50°C for 3 weeks.

## Description

### TECHNICAL FIELD

The present application relates to the technical field of biological products, and in particular to a freeze-drying excipient, a freeze-drying preservation solution, and use thereof.

### BACKGROUND

Alkaline phosphatase (ALP) is widely distributed in animals, plants, and microorganisms, has a molecular weight of approximately 86 KD, and is a zinc-containing homodimeric protein enzyme. ALP enzymes used in the field of *in vitro* diagnostics are mainly isolated from calf intestinal mucosa and *Escherichia coli*. In the field of clinical testing, such as enzyme-linked immunoassays and enzymatic chemiluminescent immunoassays, ALP enzymes and its conjugates are important components of diagnostic reagents, and their stability is a crucial factor affecting reagent performance. ALP enzymes exhibit poor thermal stability and are highly sensitive to temperature. Under elevated-temperature conditions, the activity of the ALP enzymes decreases rapidly.

In the field of in vitro diagnostics, in order to improve the stability of reagents, reagents are generally freeze-dried by in-situ freeze-drying. In-situ freeze-drying refers to a process in which a liquid is placed in a packaging container and directly freeze-dried, and is also referred to as freeze-drying in situ. However, the in-situ freeze-drying process is difficult to scale up for mass production and dispensing. Preparation of freeze-dried microspheres has become a common process for preparing freeze-dried reagents in recent years, during which a liquid diagnostic reagent is dropped into liquid nitrogen by a high-precision liquid dispenser and rapidly frozen into microspheres, followed by removal of excess liquid through an appropriate freeze-drying process. The freeze-dried microspheres can maximally preserve the activity of enzymes and proteins, and have a loose structure with excellent re-dissolving properties, and can be rapidly dissolved upon contact with a liquid.

At present, there are a wide variety of commercial liquid stabilizers for the ALP enzymes. Most claim that the ALP enzymes can be preserved for up to one year under conditions of 2 °C to 8 °C, but cannot preserve the ALP enzymes under room temperature or elevated-temperature conditions. For large in vitro diagnostic testing platforms in tertiary hospitals, storage of reagents at 2 °C to 8 °C is acceptable. However, in some small hospitals or remote areas with limited economic resources and low daily testing volumes, the low-temperature storage condition of 2°C to 8°C greatly limits the application scenarios thereof and increases the transportation cost. With the development of in vitro diagnostic technologies such as Point of Care Testing (POCT), the requirements of users for application scenarios have become increasingly higher, and there is an urgent need to develop reagents that can be preserved and used under room temperature conditions.

### SUMMARY

In view of this, one or more embodiments of the present application provide a freeze-drying excipient, a freeze-drying preservation solution, and use thereof, which can preserve the ALP enzymes under elevated-temperature conditions. The technical solutions are as follows.

According to a first aspect of the present application, a freeze-drying excipient is provided, including a saccharide, an amino acid, and an auxiliary agent in a mass ratio of (4-20):(2-12):(3-15).

The auxiliary agent includes one or more selected from the group consisting of polyvinylpyrrolidone, polyethylene glycol, mannitol, and bovine serum albumin.

In an embodiment, the mass ratio of the saccharide, the amino acid, and the auxiliary agent is (4-10):(4-8):(3-12).

In an embodiment, the saccharide includes one or more selected from the group consisting of trehalose, lactose, sucrose, and inulin.

In an embodiment, the amino acid includes one or more selected from the group consisting of alanine, glycine, leucine, lysine, valine, and serine.

In an embodiment, the polyvinylpyrrolidone includes one or more selected from the group consisting of polyvinylpyrrolidone K10, polyvinylpyrrolidone K20, polyvinylpyrrolidone K30, polyvinylpyrrolidone K40, polyvinylpyrrolidone K50, polyvinylpyrrolidone K60, polyvinylpyrrolidone K70, polyvinylpyrrolidone K80, and polyvinylpyrrolidone K90.

In an embodiment, the polyethylene glycol includes one or more selected from the group consisting of polyethylene glycol 600, polyethylene glycol 1000, polyethylene glycol 2000, polyethylene glycol 3000, polyethylene glycol 4000, polyethylene glycol 6000, polyethylene glycol 8000, polyethylene glycol 10000, and polyethylene glycol 20000.

According to a second aspect of the present application, a freeze-drying preservation solution is provided, including a freeze-drying stock solution and the freeze-drying excipient as described above.

Based on a total volume of the freeze-drying stock solution, a concentration of the saccharide is 4% (w/v) to 20% (w/v), a concentration of the amino acid is 2% (w/v) to 12% (w/v), and a concentration of the auxiliary agent is 3% (w/v) to 15% (w/v).

In an embodiment, the freeze-drying preservation solution satisfies at least one of the following conditions (1) to (4):
(1) based on the total volume of the freeze-drying stock solution, the concentration of the polyvinylpyrrolidone is 3% (w/v) to 15% (w/v);
(2) based on the total volume of the freeze-drying stock solution, the concentration of the mannitol is 3% (w/v) to 15% (w/v);
(3) based on the total volume of the freeze-drying stock solution, the concentration of the polyethylene glycol is 3% (w/v) to 15% (w/v); and
(4) based on the total volume of the freeze-drying stock solution, the concentration of the bovine serum albumin is 0.5% (w/v) to 10% (w/v).

In an embodiment, the freeze-drying stock solution includes a MES buffer, magnesium chloride hexahydrate, Triton X-100, fish skin gelatin, calcium chloride dihydrate, and zinc chloride. The freeze-drying preservation solution satisfies at least one of the following conditions (1) to (5):
(1) based on a volume of the MES buffer, a concentration of the magnesium chloride hexahydrate is 0.1 mM to 2 mM;
(2) based on the volume of the MES buffer, a concentration of the Triton X-100 is 0.4% (w/v) to 2% (w/v);
(3) based on the volume of the MES buffer, a concentration of the fish skin gelatin is 0.2% (w/v) to 3% (w/v);
(4) based on the volume of the MES buffer, a concentration of the calcium chloride dihydrate is 1 mM to 12 mM; and
(5) based on the volume of the MES buffer, a concentration of the zinc chloride is 0.1 mM to 3 mM.

According to a third aspect of the present application, a kit is provided, including alkaline phosphatase and the freeze-drying preservation solution as described above.

According to a fourth aspect of the present application, use of the freeze-drying excipient as described above, the freeze-drying preservation solution as described above, or the kit as described above in preparing freeze-dried microspheres is provided.

Compared with the conventional technology, the present application has the following beneficial effects:
In the present application, the saccharide and the amino acid at specific concentrations and the auxiliary agent of a specific type are selected for the freeze-drying excipient. The components cooperate with each other and act synergistically, so that when the prepared freeze-drying preservation solution is used for preserving the ALP enzyme, it can be stored in a sealed manner for more than 18 months under room temperature conditions, and maintains an enzyme activity retention rate above 87% after storage under an elevated-temperature condition of 50°C for 3 weeks. In addition, the freeze-dried microspheres prepared using the freeze-drying preservation solution of the present application are characterized by being convenient for production and packaging.

### BRIEF DESCRIPTION OF THE DRAWINGS

To describe the technical solutions in the specific embodiments of the present application or in the prior art more clearly, the following briefly describes the accompanying drawings required for describing the specific embodiments or the prior art. Apparently, the accompanying drawings in the following description show merely some embodiments of the present application, and for a person of ordinary skill in the art, other drawings may be derived from these accompanying drawings without creative efforts.

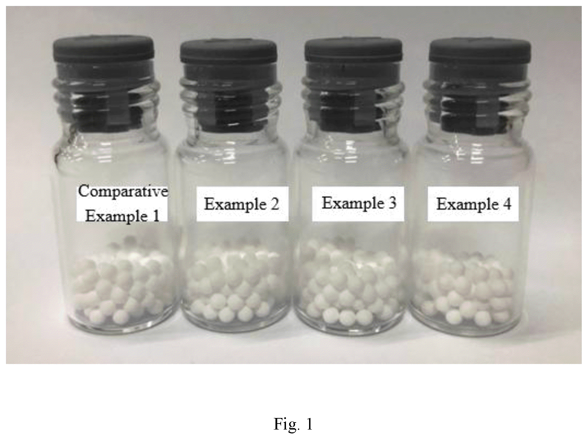

FIG. 1 shows the appearance morphology diagram of the freeze-dried microspheres prepared in Example 2 to Example 4 of the present application and Comparative Example 1 after storage under room-temperature conditions for 18 months.

### DETAILED DESCRIPTION

In order to make the above objects, features, and advantages of the present application more apparent and readily understood, the specific embodiments of the present application are described in detail below. Numerous specific details are set forth in the following description in order to facilitate a full understanding of the present application. However, the present application may be implemented in many other forms different from those described herein, and those skilled in the art may make similar modifications without departing from the concept of the present application. Therefore, the present application is not limited to the specific embodiments disclosed below.

Unless otherwise defined, all technical and scientific terms used herein have the same meanings as commonly understood by those skilled in the art to which the present application pertains. The terms used herein in the description of the present application are only for the purpose of describing specific embodiments, and are not intended to limit the present application. Unless otherwise specifically indicated, various raw materials, reagents, instruments, and equipment used in the present application are commercially available or can be prepared by conventional methods.

In a first aspect of the present application, a freeze-drying excipient is provided, including a saccharide, an amino acid, and an auxiliary agent in a mass ratio of (4-20):(2-12):(3-15).

The auxiliary agent includes one or more selected from the group consisting of polyvinylpyrrolidone, polyethylene glycol, mannitol, and bovine serum albumin.

Different freeze-drying excipients have a great influence on the appearance, stability, hardness, and other properties of freeze-dried microspheres. Therefore, selecting an appropriate freeze-drying excipient is of great importance for the freeze-dried microspheres.

As an important freeze-drying protectant, the saccharide can protect the ALP enzyme from loss of activity during the freezing and drying processes. Furthermore, when the saccharide is combined with the above auxiliary agent, the appearance, resistance to elevated temperatures, and hardness of the freeze-dried microspheres can be significantly improved. Moreover, the addition of the amino acid at a specific concentration can significantly improve the surface depression of the freeze-dried microspheres, so that the surfaces of the freeze-dried microspheres become smoother.

In some embodiments, in the above freeze-drying excipient, the mass ratio of the saccharide, the amino acid, and the auxiliary agent is (4-10):(4-8):(3-12). Optionally, the mass ratio of the saccharide, the amino acid, and the auxiliary agent is (4-8):(6-8):(3-8). Further optionally, the mass ratio of the saccharide, the amino acid, and the auxiliary agent is 8:8:3.

In some specific embodiments, the saccharide includes one or more selected from the group consisting of trehalose, lactose, sucrose, and inulin.

In some specific embodiments, the amino acid includes one or more selected from the group consisting of alanine, glycine, leucine, lysine, valine, and serine.

In some specific embodiments, the polyvinylpyrrolidone includes one or more selected from the group consisting of polyvinylpyrrolidone K10 (PVP K10), polyvinylpyrrolidone K20 (PVP K20), polyvinylpyrrolidone K30 (PVP K30), polyvinylpyrrolidone K40 (PVP K40), polyvinylpyrrolidone K50 (PVP K50), polyvinylpyrrolidone K60 (PVP K60), polyvinylpyrrolidone K70 (PVP K70), polyvinylpyrrolidone K80 (PVP K80), and polyvinylpyrrolidone K90 (PVP K90).

In an optional example, the polyvinylpyrrolidone is selected from polyvinylpyrrolidone K40.

In some specific embodiments, the polyethylene glycol includes one or more selected from the group consisting of polyethylene glycol 600, polyethylene glycol 1000, polyethylene glycol 2000, polyethylene glycol 3000, polyethylene glycol 4000, polyethylene glycol 6000, polyethylene glycol 8000, polyethylene glycol 10000, and polyethylene glycol 20000.

In an optional example, the polyethylene glycol is selected from polyethylene glycol 20000.

It can be understood that the above freeze-drying excipient may be pre-prepared or freshly prepared, and the above mass ratio refers to the mass ratio of the three components when they are present in combination.

In a second aspect of the present application, a freeze-drying preservation solution is provided, including a freeze-drying stock solution and the freeze-drying excipient according to any one of the above embodiments. Based on the total volume of the freeze-drying stock solution, the concentration of the saccharide is 4% (w/v) to 20% (w/v), the concentration of the amino acid is 2% (w/v) to 12% (w/v), and the concentration of the auxiliary agent is 3% (w/v) to 15% (w/v).

In some embodiments, the concentration of the saccharide is 4% (w/v) to 10% (w/v), the concentration of the amino acid is 4% (w/v) to 8% (w/v), and the concentration of the auxiliary agent is 3% (w/v) to 12% (w/v).

In some embodiments, based on the total volume of the freeze-drying stock solution, the concentration of the polyvinylpyrrolidone is 3% (w/v) to 15% (w/v). Optionally, the concentration of the polyvinylpyrrolidone is 3% (w/v).

In some embodiments, based on the total volume of the freeze-drying stock solution, the concentration of the mannitol is 3% (w/v) to 15% (w/v). Optionally, the concentration of the mannitol is 12% (w/v).

In some embodiments, based on the total volume of the freeze-drying stock solution, the concentration of the polyethylene glycol is 3% (w/v) to 15% (w/v). Optionally, the concentration of the polyethylene glycol is 3% (w/v).

In some embodiments, based on the total volume of the freeze-drying stock solution, the concentration of the bovine serum albumin is 0.5% (w/v) to 10% (w/v). Optionally, the concentration of the bovine serum albumin is 4% (w/v).

In some embodiments, the freeze-drying stock solution includes MES buffer, magnesium chloride hexahydrate, Triton X-100, fish skin gelatin, calcium chloride dihydrate, and zinc chloride.

In some specific embodiments, the concentration of the MES buffer is 40 mM to 60 mM. Optionally, the concentration of the MES buffer is 45 mM to 55 mM. Further optionally, the concentration of the MES buffer is 50 mM.

In some embodiments, the pH value of the MES buffer is 5.8 to 6.2. Optionally, the pH value of the MES buffer is 5.9 to 6.1. Further optionally, the pH value of the MES buffer is 6.0.

In some specific embodiments, based on the volume of the MES buffer, the concentration of the magnesium chloride hexahydrate is 0.1 mM to 2 mM. Optionally, the concentration of the magnesium chloride hexahydrate is 0.4 mM to 0.6 mM. Further optionally, the concentration of the magnesium chloride hexahydrate is 0.5 mM.

In some specific embodiments, based on the volume of the MES buffer, the concentration of the Triton X-100 is 0.4% (w/v) to 2% (w/v). Optionally, the concentration of the Triton X-100 is 0.4% (w/v) to 0.6% (w/v). Further optionally, the concentration of the Triton X-100 is 0.5% (w/v).

In some specific embodiments, based on the volume of the MES buffer, the concentration of the fish skin gelatin is 0.2% (w/v) to 3% (w/v). Optionally, the concentration of the fish skin gelatin is 1.8% (w/v) to 2.2% (w/v). Further optionally, the concentration of the fish skin gelatin is 2% (w/v).

In some specific embodiments, based on the volume of the MES buffer, the concentration of the calcium chloride dihydrate is 1 mM to 12 mM. Optionally, the concentration of the calcium chloride dihydrate is 8 mM to 11 mM. Further optionally, the concentration of the calcium chloride dihydrate is 10 mM.

In some specific embodiments, based on the volume of the MES buffer, the concentration of the zinc chloride is 0.1 mM to 3 mM. Optionally, the concentration of the zinc chloride is 0.5 mM to 0.7 mM. Further optionally, the concentration of the zinc chloride is 0.6 mM.

In the above freeze-drying preservation solution of the present application, the saccharide, the amino acid, and the auxiliary agent of specific types are mixed at specific concentrations, so that the prepared freeze-drying preservation solution can maintain the enzyme activity retention rate of the alkaline phosphatase under room temperature conditions and even under elevated-temperature conditions (e.g., 50 °C). Furthermore, the freeze-dried microspheres prepared therefrom can maintain a smooth surface without depression and have moderate hardness under both room-temperature and elevated-temperature conditions, and do not shrink or melt under elevated-temperature conditions.

In addition, the freeze-drying preservation solution of the present application has the advantages of a simple formulation and low cost.

In a third aspect of the present application, a kit is provided, including the alkaline phosphatase and the freeze-drying preservation solution according to any one of the above embodiments.

It can be understood that the above freeze-drying preservation solution may be a pre-prepared freeze-drying preservation solution or a freshly prepared freeze-drying preservation solution.

In some embodiments, the above kit further includes an anti-human IgE antibody.

In some specific embodiments, the alkaline phosphatase is used to label the anti-human IgE antibody. Optionally, based on the volume of the MES buffer, the concentration of the anti-human IgE antibody labeled with the alkaline phosphatase is 0.7 µg/mL to 0.9 µg/mL. Further optionally, the concentration of the anti-human IgE antibody labeled with the alkaline phosphatase is 0.75 µg/mL to 0.85 µg/mL. Further optionally, the concentration of the anti-human IgE antibody labeled with the alkaline phosphatase is 0.8 µg/mL.

In the present application, unless otherwise specified, the contents of the saccharide, the amino acid, and the auxiliary agent are based on the relative contents thereof when the three components are mixed for use. However, the form of the freeze-drying excipient provided by the present application is not limited thereto. The three components may be provided separately, as long as the three components can be mixed into the same system during use.

In a fourth aspect of the present application, use of the above freeze-drying excipient, the above freeze-drying preservation solution, or the kit in preparing freeze-dried microspheres is provided.

The freeze-dried microspheres prepared by using the above freeze-drying preservation solution of the present application have at least the following advantages:
(1) The problem of unstable storage of the ALP enzyme and its conjugates under room temperature conditions and elevated-temperature conditions is alleviated: the freeze-dried microspheres of the present application can be placed under room-temperature conditions for 18 months while maintaining the activity of the ALP enzyme at 90% or more.
(2) The appearance of the freeze-dried microspheres is optimized: the freeze-dried microspheres have a smooth surface without depression, have moderate hardness, and cannot shrink or melt under a destructive elevated-temperature condition of 50 °C.
(3) The in-use stability of the freeze-dried microspheres is improved: under conditions of 30 °C and 60% RH, the appearance of the freeze-dried microspheres shows no significant change.
(4) Packaging into single-use units is facilitated, thereby reducing repeated freeze-thaw cycles during use, and the large-scale production can be achieved.

The present application will be further described below with reference to specific examples and comparative examples, which, however, should not be construed as limiting the protection scope of the present application.

### Example 1: Evaluation of the effects of different freeze-drying excipients on the appearance properties of microspheres

### (1) Preparation of the freeze-drying preservation solution

### a) Preparation of the freeze-drying stock solution

Based on a MES buffer having a pH value of 6.0 and a concentration of 50 mM, 0.5 mM magnesium chloride hexahydrate, 0.5% (w/v) Triton X-100, 2% (w/v) fish skin gelatin, 10 mM calcium chloride dihydrate, 0.6 mM zinc chloride, and 0.8 µg/mL of a conjugate of an anti-human IgE labeled with the ALP enzyme were provided and mixed uniformly to prepare an ALP enzyme conjugate stock solution.

The ALP enzyme used in this example was purchased from BBI Solutions, with product No. ALPI12G.

### b) Preparation of the freeze-drying excipients

Based on the total volume of the freeze-drying stock solution prepared in step a), the raw materials for the freeze-drying excipients shown in Table 1 were provided and mixed uniformly to prepare 12 freeze-drying excipients having different formulations.

**Table 1**

| Group No. | Freeze-drying excipients |
|---|---|
| 1 | 10% (w/v) trehalose |
| 2 | 10% (w/v) lactose |
| 3 | 10% (w/v) sucrose |
| 4 | 10% (w/v) inulin |
| 5 | 10% (w/v) PVP K40 |
| 6 | 10% (w/v) mannitol |
| 7 | 10% (w/v) PEG 20000 |
| 8 | 10% (w/v) mannitol +6% (w/v) trehalose |
| 9 | 10% (w/v) PEG 20000+6% (w/v) trehalose |
| 10 | 4%(w/v) PVP K40+6% (w/v) trehalose |
| 11 | 10% (w/v) trehalose +4% (w/v) BSA |
| 12 | 10% (w/v) trehalose +4% (w/v) BSA+4% (w/v) glycine |

c) The 12 freeze-drying excipients prepared in step b) were each added to the ALP enzyme conjugate stock solution prepared in step a) to prepare freeze-drying preservation solutions.

### (2) Preparation of the freeze-dried microspheres

An IVEK Digispense 3009 system was used, with the liquid dispensing volume adjusted to 5 µL. The freeze-drying preservation solutions were dropped into a separator storing liquid nitrogen to form frozen microspheres. The frozen microspheres were then transferred into a vacuum freeze dryer pre-cooled to -50 °C for freeze-drying. The freeze-drying parameters are shown in Table 2.

**Table 2**

| Freeze-drying procedure | Shelf temperature/°C | Cold trap temperature/°C | Vacuum/mbar | Time/h |
|---|---|---|---|---|
| Pre-freezing | -50 | -70 | off | 3 |
| | -30 | -70 | off | 2 |
| | -50 | -70 | off | 2 |
| Primary drying | -50 | -70 | 0.4 | 0.5 |
| | -50 | -70 | 0.1 | 1 |
| | -30 | -70 | 0.1 | 20 |
| Secondary drying | 20 | -70 | 0.05 | 7 |

### (3) Packaging of the freeze-dried microspheres

After completion of the freeze-drying process, the freeze-dried microspheres were packaged in a dry room at a humidity of less than 40% and a temperature of about 25 °C.

### (4) Evaluation of the properties of the freeze-dried microspheres

A portion of the packaged freeze-dried microspheres was placed in a drying oven at a humidity of less than 40% and a temperature of 50 °C. After accelerated degradation at the elevated temperature for 11 days, the dimensional change of the freeze-dried microspheres was measured using a vernier caliper, and the appearance of the freeze-dried microspheres was observed under a stereomicroscope. The hardness property was evaluated by a drop test. When the freeze-dried microspheres were dropped from a height of 1 m onto a laboratory bench, no fracture and no powder shedding was rated as "high hardness"; no fracture but powder shedding was rated as "moderately high hardness"; and fracture was rated as "low hardness".

The appearance characteristics with different freeze-drying excipients after accelerated degradation at 50 °C for 11 days are shown in Table 3, wherein "-" indicates the presence of shrinkage or melting, surface depression, or non-uniform size, and "+" indicates the absence of shrinkage or melting, a smooth surface without depression, and uniform size. When the appearance was "-" or the hardness was "low", the appearance was considered unqualified.

**Table 3**

| Group No. | Freeze-drying excipients | Shrinkage or melting | Surface depression | Size uniformity | Hardness | Appearance |
|---|---|---|---|---|---|---|
| 1 | 10% (w/v) trehalose | - | - | - | high | unqualified |
| 2 | 10% (w/v) lactose | - | - | - | high | unqualified |
| 3 | 10% (w/v) sucrose | - | - | - | high | unqualified |
| 4 | 10% (w/v) inulin | - | - | - | high | unqualified |
| 5 | 10% (w/v) PVP K40 | + | + | + | moderately high | qualified |
| 6 | 10% (w/v) mannitol | + | + | + | low | unqualified |
| 7 | 10% (w/v) PEG 20000 | + | + | + | low | unqualified |
| 8 | 10% (w/v) mannitol +6% (w/v) trehalose | + | + | + | moderately high | qualified |
| 9 | 10% (w/v) PEG 20000+6% (w/v) trehalose | + | + | + | moderately high | qualified |
| 10 | 4%(w/v) PVP K40+6% (w/v) trehalose | + | + | + | high | qualified |
| 11 | 10% (w/v) trehalose +4% (w/v) BSA | + | + | + | high | qualified |
| 12 | 10% (w/v) trehalose +4% (w/v) BSA+4% (w/v) glycine | + | + | + | high | qualified |

As can be seen from Groups 1 to 4 in Table 3, when saccharides alone were added as the freeze-drying excipients, the freeze-dried microspheres underwent shrinkage or even melting after accelerated degradation at 50 °C for 11 days. As can be seen from Groups 8 to 11 and Group 1, in order to improve the resistance of the saccharides to elevated temperatures, combining trehalose with mannitol, PEG 20000, PVP K40, or BSA, respectively, can significantly improve the appearance and resistance to elevated temperatures of the freeze-dried microspheres. As can be seen from Groups 6 to 9, on the basis of mannitol or PEG 20000, the saccharides added at appropriate concentrations can significantly improve the hardness of the freeze-dried microspheres. As can be seen from Groups 11 and 12, the amino acid added at an appropriate amount can improve surface depression of the freeze-dried microspheres, thereby making the surface of the freeze-dried microspheres smoother.

The above results indicate that using a mixture of the saccharide, the freeze-drying auxiliary agent, and the amino acid at specific concentrations as the freeze-drying excipients can effectively improve the resistance of the freeze-dried microspheres to elevated temperatures (50°C), while maintaining a smooth appearance surface, uniform size, and good hardness.

### Example 2

The procedure was substantially the same as that in Example 1, except that the formulation of the freeze-drying excipient was different. Based on the total volume of the freeze-drying stock solution, the freeze-drying excipient included 3% (w/v) PVP K40, 8% (w/v) trehalose, and 8% (w/v) alanine.

The prepared freeze-dried microspheres were placed in a drying oven at 50°C for accelerated degradation for three weeks, and their properties before and after the accelerated degradation were evaluated.

Evaluation of dissolution rate: Three freeze-dried microspheres were each placed in a well of an elisa plate, and 30 µL of ultrapure water was added to each well. Each type of the freeze-dried microspheres was tested three times, and the average time for complete dissolution of each type of the freeze-dried microsphere was recorded.

Evaluation of enzyme activity retention rate: After ten freeze-dried microspheres were dissolved in 100 µL of ultrapure water, the activity retention rate of alkaline phosphatase during dissolution (the ratio of the activity after the accelerated test at 50°C to the activity after dry storage at 4°C) was determined using a Mindray automatic biochemical analyzer and a matching alkaline phosphatase assay kit. As shown in Table 4, the results indicate that the freeze-dried microspheres exhibited good enzyme activity retention rate, appearance characteristic, dissolution rate, and moisture content under the elevated-temperature condition of 50°C.

Test of in-use stability: the microspheres were placed under conditions of 30°C and 60% RH for 3 hours, after which no obvious shrinkage was observed on the surfaces of microspheres.

**Table 4**

| Performance indexes | After 1 week of accelerated degradation | After 2 weeks of accelerated degradation | After 3 weeks of accelerated degradation |
|---|---|---|---|
| Enzyme activity retention rate | 85% | 82% | 77% |
| Shrinkage or melting | + | + | + |
| Surface depression | + | + | + |
| Uniform Size | + | + | + |
| Hardness | + | + | + |
| Appearance | qualified | qualified | qualified |
| Dissolution rate | 1s | 1s | 1s |
| Moisture content | 1.4% | 1.4% | 1.2% |

### Example 3

The procedure was substantially the same as that in Example 1, except that the formulation of the freeze-drying excipient was different. Based on the total volume of the freeze-drying stock solution, the freeze-drying excipient included 3% (w/v) PEG 20000, 8% (w/v) inulin, and 8% (w/v) glycine.

The prepared freeze-dried microspheres were placed in a drying oven at 50°C for accelerated degradation for three weeks, and their properties before and after the accelerated degradation were evaluated. As shown in Table 5, the results indicate that the enzyme activity retention rate of the freeze-dried microspheres remained above 90% after 3 weeks of accelerated degradation under the elevated-temperature condition of 50°C.

Test of in-use stability: the microspheres were placed under conditions of 30 °C and 60% RH for 3 hours, after which no obvious shrinkage was observed on the surfaces of microspheres.

**Table 5**

| Performance indexes | After 1 week of accelerated degradation | After 2 weeks of accelerated degradation | After 3 weeks of accelerated degradation |
|---|---|---|---|
| Enzyme activity retention rate | 95% | 95% | 92% |
| No shrinkage or melting | + | + | + |
| Smooth surface without depression | + | + | + |
| Uniform Size | + | + | + |
| Hardness | + | + | + |
| Appearance | qualified | qualified | qualified |
| Dissolution rate | 1s | 1s | 1s |
| Moisture content | 1.4% | 1.2% | 1.2% |

### Example 4

The procedure was substantially the same as that in Example 1, except that the formulation of the freeze-drying excipient was different. Based on the total volume of the freeze-drying stock solution, the freeze-drying excipient included 12% (w/v) mannitol, 4% (w/v) inulin, and 4% (w/v) alanine.

The prepared freeze-dried microspheres were placed in a drying oven at 50°C for accelerated degradation for three weeks, and their properties before and after the accelerated degradation were evaluated. As shown in Table 6, the results indicate that the freeze-dried microspheres exhibited good enzyme activity retention rate, appearance characteristic, dissolution rate, and moisture content under the elevated-temperature condition of 50°.

Test of in-use stability: the microspheres were placed under conditions of 30°C and 60% RH for 3 hours, after which no obvious shrinkage was observed on the surfaces of microspheres.

**Table 6**

| Performance indexes | After 1 week of accelerated degradation | After 2 weeks of accelerated degradation | After 3 weeks of accelerated degradation |
|---|---|---|---|
| Enzyme activity retention rate | 92% | 90% | 87% |
| No shrinkage or melting | + | + | + |
| Smooth surface without depression | + | + | + |
| Uniform Size | + | + | + |
| Hardness | + | + | + |
| Appearance | qualified | qualified | qualified |
| Dissolution rate | 1s | 1s | 1s |
| Moisture content | 1.7% | 1.3% | 1.2% |

### Example 5

This example was substantially the same as Example 4, except that the formulation of the freeze-drying excipients was different. Based on the total volume of the freeze-drying stock solution, the freeze-drying excipients included 12% (w/v) mannitol, 4% (w/v) sucrose, and 4% (w/v) alanine. The performance results are shown in Table 7.

**Table 7**

| Performance indexes | One week | Two weeks | Three weeks |
|---|---|---|---|
| Enzyme activity retention rate | 87% | 86% | 86% |
| No shrinkage or melting | + | + | + |
| Smooth surface without depression | + | + | + |
| Uniform Size | + | + | + |
| Hardness | + | + | + |
| Appearance | qualified | qualified | qualified |
| Dissolution rate | 1s | 1s | 1s |
| Moisture content | 1.8% | 1.6% | 1.6% |

### Comparative Example 1:

The procedure was substantially the same as that in Example 1, except that the formulation of the freeze-drying excipient was different. Based on the total volume of the freeze-drying stock solution, the freeze-drying excipient included only 12% (w/v) PVP K40.

The performance results are shown in Table 8. When only PVP K40 was added as the freeze-drying excipient, the appearance of the freeze-dried microspheres was qualified, but the enzyme activity retention performance was reduced compared with those of the Examples. In addition, after the freeze-dried microspheres were placed under conditions of 30 °C and 60% RH for 3 hours, obvious shrinkage appeared on the surfaces of the freeze-dried microspheres.

**Table 8**

| Performance indexes | One week | Two weeks | Three weeks |
|---|---|---|---|
| Enzyme activity retention rate | 85% | 82% | 77% |
| No shrinkage or melting | + | + | + |
| Smooth surface without depression | + | + | + |
| Uniform Size | + | + | + |
| Hardness | + | + | + |
| Appearance | qualified | qualified | qualified |
| Dissolution rate | 1s | 1s | 1s |
| Moisture content | 1.4% | 1.4% | 1.2% |

### Comparative Example 2:

The procedure was substantially the same as that in Example 2, except that the formulation of the freeze-drying excipient was different. Based on the total volume of the freeze-drying stock solution, the freeze-drying excipient included 8% (w/v) trehalose, 1% (w/v) alanine, and 3% (w/v) PVP K40.

The performance results are shown in Table 9. Compared with Example 2, the enzyme activity retention rate of the freeze-dried microspheres of Comparative Example 2 was reduced, and depressions appeared on the surfaces of the microspheres.

**Table 9**

| Performance indexes | One week | Two weeks | Three weeks |
|---|---|---|---|
| Enzyme activity retention rate | 80% | 78% | 75% |
| No shrinkage or melting | + | + | + |
| Smooth surface without depression | - | - | - |
| Uniform Size | + | + | + |
| Hardness | + | + | + |
| Appearance | unqualified | unqualified | unqualified |
| Dissolution rate | 1s | 1s | 1s |
| Moisture content | 1.5% | 1.4% | 1.4% |

### Comparative Example 3:

The procedure was substantially the same as that in Example 2, except that the formulation of the freeze-drying excipient was different. Based on the total volume of the freeze-drying stock solution, the freeze-drying excipient included 8% (w/v) trehalose, 8% (w/v) alanine, and 1% (w/v) PVP K40.

The performance results are shown in Table 10. Compared with Example 2, the enzyme activity retention rate of the freeze-dried microspheres of Comparative Example 3 was reduced, and the surfaces of the microspheres began to shrink or melt after two weeks.

**Table 10**

| Performance indexes | One week | Two weeks | Three weeks |
|---|---|---|---|
| Enzyme activity retention rate | 83% | 77% | 75% |
| No shrinkage or melting | + | - | - |
| Smooth surface without depression | + | + | + |
| Uniform Size | + | + | + |
| Hardness | + | + | + |
| Appearance | qualified | qualified | qualified |
| Dissolution rate | 1s | 1s | 1s |
| Moisture content | 1.3% | 1.3% | 1.2% |

### Performance Test

In order to verify the effect of the above freeze-drying excipients on the storage stability of the ALP enzyme and its conjugate under room temperature conditions, the freeze-dried microspheres prepared in Examples 2 to 4 and Comparative Example 1 were each placed in sealed glass bottles containing a desiccant, and were each tested for the enzyme activity retention rate and appearance after storage under room temperature conditions (20°C to 30°C) for 18 months. The results are shown in Table 11, and the appearances are shown in FIG. 1.

**Table 11**

| Source of the freeze-dried microspheres | Enzyme activity retention rate | Appearance |
|---|---|---|
| Example 2 | 91% | qualified |
| Example 3 | 96% | qualified |
| Example 4 | 90% | qualified |
| Comparative Example 1 | 85% | qualified |

In conclusion, it can be seen that the freeze-dried microspheres prepared using the freeze-drying preservation solution having the specific formulation of the present application can maintain stability, good hardness, and a qualified appearance under elevated-temperature conditions, and still have a good enzyme activity retention rate after storage under room temperature conditions for 18 months. The formulation of the freeze-drying preservation solution of the present application provides a new approach for overcoming the limitations on the application of alkaline phosphatase under room temperature and scenarios.

The technical features of the above-mentioned embodiments can be combined arbitrarily. In order to make the description concise, not all possible combinations of the technical features in the embodiments are described. However, as long as there is no contradiction in the combination of these technical features, the combinations should be considered as in the scope of the present disclosure.

The above-described embodiments are only several implementations of the present disclosure, and the descriptions are relatively specific and detailed, but they should not be construed as limiting the scope of the present disclosure. It should be understood by those of ordinary skill in the art that various modifications and improvements can be made without departing from the concept of the present disclosure, and all fall within the protection scope of the present disclosure. Therefore, the patent protection of the present disclosure shall be defined by the appended claims.

## Claims

1. A freeze-drying excipient, **characterized by** comprising a saccharide, an amino acid, and an auxiliary agent in a mass ratio of (4-20):(2-12):(3-15);
wherein the auxiliary agent comprises one or more selected from the group consisting of polyvinylpyrrolidone, polyethylene glycol, mannitol, and bovine serum albumin.

2. The freeze-drying excipient according to claim 1, **characterized in that** the mass ratio of the saccharide, the amino acid, and the auxiliary agent is (4-10):(4-8):(3-12).

3. The freeze-drying excipient according to any one of claims 1 to 2, **characterized in that** the saccharide comprises one or more selected from the group consisting of trehalose, lactose, sucrose, and inulin.

4. The freeze-drying excipient according to any one of claims 1 to 2, **characterized in that** the amino acid comprises one or more selected from the group consisting of alanine, glycine, leucine, lysine, valine, and serine.

5. The freeze-drying excipient according to any one of claims 1 to 2, **characterized in that** the polyvinylpyrrolidone comprises one or more selected from the group consisting of polyvinylpyrrolidone K10, polyvinylpyrrolidone K20, polyvinylpyrrolidone K30, polyvinylpyrrolidone K40, polyvinylpyrrolidone K50, polyvinylpyrrolidone K60, polyvinylpyrrolidone K70, polyvinylpyrrolidone K80, and polyvinylpyrrolidone K90.

6. The freeze-drying excipient according to any one of claims 1 to 2, **characterized in that** the polyethylene glycol comprises one or more selected from the group consisting of polyethylene glycol 600, polyethylene glycol 1000, polyethylene glycol 2000, polyethylene glycol 3000, polyethylene glycol 4000, polyethylene glycol 6000, polyethylene glycol 8000, polyethylene glycol 10000, and polyethylene glycol 20000.

7. A freeze-drying preservation solution, **characterized by** comprising a freeze-drying stock solution and the freeze-drying excipient according to any one of claims 1 to 6;
wherein based on a total volume of the freeze-drying stock solution, a concentration of the saccharide is 4% (w/v) to 20% (w/v), a concentration of the amino acid is 2% (w/v) to 12% (w/v), and a concentration of the auxiliary agent is 3% (w/v) to 15% (w/v).

8. The freeze-drying preservation solution according to claim 7, **characterized in that** the freeze-drying preservation solution satisfies at least one of the following conditions (1) to (4):
(1) based on the total volume of the freeze-drying stock solution, the concentration of the polyvinylpyrrolidone is 3% (w/v) to 15% (w/v);
(2) based on the total volume of the freeze-drying stock solution, the concentration of the mannitol is 3% (w/v) to 15% (w/v);
(3) based on the total volume of the freeze-drying stock solution, the concentration of the polyethylene glycol is 3% (w/v) to 15% (w/v); and
(4) based on the total volume of the freeze-drying stock solution, the concentration of the bovine serum albumin is 0.5% (w/v) to 10% (w/v).

9. The freeze-drying preservation solution according to any one of claims 7 to 8, **characterized in that** the freeze-drying stock solution comprises a MES buffer, magnesium chloride hexahydrate, Triton X-100, fish skin gelatin, calcium chloride dihydrate, and zinc chloride; and the freeze-drying preservation solution satisfies at least one of the following conditions (1) to (5):
(1) based on a volume of the MES buffer, a concentration of the magnesium chloride hexahydrate is 0.1 mM to 2 mM;
(2) based on the volume of the MES buffer, a concentration of the Triton X-100 is 0.4% (w/v) to 2% (w/v);
(3) based on the volume of the MES buffer, a concentration of the fish skin gelatin is 0.2% (w/v) to 3% (w/v);
(4) based on the volume of the MES buffer, a concentration of the calcium chloride dihydrate is 1 mM to 12 mM; and
(5) based on the volume of the MES buffer, a concentration of the zinc chloride is 0.1 mM to 3 mM.

10. A kit, **characterized by** comprising alkaline phosphatase and the freeze-drying preservation solution according to any one of claims 7 to 9.

11. Use of the freeze-drying excipient according to any one of claims 1 to 6, the freeze-drying preservation solution according to any one of claims 7 to 9, or the kit according to claim 10 in preparing freeze-dried microspheres.
